Europäisches Patentamt

European Patent Office

Office européen des brevets

(19)

(11) **EP 1 434 790 B1**

(12) **EUROPEAN PATENT SPECIFICATION**

(45) Date of publication and mention
of the grant of the patent:
**13.12.2006 Bulletin 2006/50**

(51) Int Cl.:
*C07K 1/14* (2006.01)　　　*C07K 1/34* (2006.01)
*C07K 14/47* (2006.01)

(21) Application number: **02757677.6**

(22) Date of filing: **11.09.2002**

(86) International application number:
**PCT/US2002/028814**

(87) International publication number:
**WO 2003/022870 (20.03.2003 Gazette 2003/12)**

(54) **CALCIUM-INFLUX INHIBITORY FACTOR AND METHOD OF ISOLATION THEREOF**

DEN CALCIUMEINSTROM HEMMENDER FAKTOR UND VERFAHREN ZU DESSEN ISOLIERUNG

FACTEUR D'INHIBITION D'AFFLUX DE CALCIUM ET METHODE D'ISOLEMENT DUDIT FACTEUR

(84) Designated Contracting States:
**AT BE BG CH CY CZ DE DK EE ES FI FR GB GR
IE IT LI LU MC NL PT SE SK TR**

(30) Priority: **12.09.2001 US 322057 P**

(43) Date of publication of application:
**07.07.2004 Bulletin 2004/28**

(73) Proprietor: **Eastern Virginia Medical School
Norfolk, VA 23507 (US)**

(72) Inventor: **BUESCHER, E., Stephen
Virginia Beach, VA 23452 (US)**

(74) Representative: **Lee, Nicholas John et al
Kilburn & Strode,
20 Red Lion Street
London WC1R 4PJ (GB)**

(56) References cited:
WO-A1-95/29933　　　　US-A- 5 595 738
US-A- 5 667 797　　　　US-B1- 6 177 550

• BUESCHER STEPHEN ET AL: "Human milk
exposure causes transient rises in intracellular
free Ca++ levels, depletion of intracellular Ca++
stores and blockade of Ca++ influx in human
polymorphonuclear leukocytes (PMN)"
PEDIATRIC RESEARCH, vol. 51, no. 4 Part 2, April
2002 (2002-04), page 273A, XP009036583 &
ANNUAL MEETING OF THE PEDIATRIC
SOCIETIES'; BALTIMORE, MD, USA; MAY 04-07,
2002 ISSN: 0031-3998

• BUESCHER STEPHEN ET AL: "Blockade of Ca++
influx by human milk factor(s) selectively affects
polymorphonuclear leukocyte functions"
PEDIATRIC RESEARCH, vol. 47, no. 4 Part 2, April
2000 (2000-04), page 390A, XP009036595 & JOINT
MEETING OF THE PEDIATRIC ACADEMIC
SOCIETIES AND THE AMERICAN ACADEMY OF
PEDIATRICS.; BOSTON, MASSACHUSETTS,
USA; MAY 12-16, 2000 ISSN: 0031-3998

• CHACON-CRUZ ENRIQUE ET AL: "Human milk
exposure blocks calcium influx into human
polymorphonuclear leukocytes" PEDIATRIC
RESEARCH, vol. 43, no. 4 PART 2, April 1998
(1998-04), page 7A, XP009036624 & ANNUAL
MEETING OF THE AMERICAN PEDIATRIC
SOCIETY AND THE SOCIETY FOR PEDIATRIC
RESEARCH; NEW ORLEANS, LOUISIANA, USA;
MAY 1-5, 1998 ISSN: 0031-3998

• CHACON-CRUZ ENRIQUE ET AL: "Human milk's
effects on releasing Ca++ from neutrophil internal
stores are G-protein mediated and membrane
potential dependent" PEDIATRIC RESEARCH,
vol. 47, no. 4 Part 2, April 2000 (2000-04), page
16A, XP009036594 & JOINT MEETING OF THE
PEDIATRIC ACADEMIC SOCIETIES AND THE
AMERICAN ACADEMY OF PEDIATRICS.;
BOSTON, MASSACHUSETTS, USA; MAY 12-16,
2000 ISSN: 0031-3998

• CHACON-CRUZ ENRIQUE ET AL: "Human milk
effects on neutrophil calcium metabolism:
Blockade of calcium influx after agonist
stimulation" PEDIATRIC RESEARCH, vol. 46, no.
2, August 1999 (1999-08), pages 200-207,
XP009036593 ISSN: 0031-3998

• BUESCHER E STEPHEN ET AL: "Human milk anti-inflammatory component contents during acute mastitis" CELLULAR IMMUNOLOGY, vol. 210, no. 2, 15 June 2001 (2001-06-15), pages 87-95, XP002296441 ISSN: 0008-8749

**Description**

Background

Polymorphonuclear leukocytes or granulocytes are a broad class of white blood cell including neutrophils, eosinophils, and basophils, which are produced in the bone marrow and defend the body against infecting organisms or foreign substances, and neutrophils are the predominant cellular component of acute inflammation. Migration of neutrophils into the afflicted area is characteristic of an inflammatory response. Neutrophils have also been shown to play a role in inflammatory disease, and "inappropriate" neutrophil activation and subsequent suppression is proposed to explain the role of neutrophils in certain diseases. For example, inappropriate activation, deactivation and probable autooxidative damage has been implicated as a mechanism of neutrophil locomotory defect in trauma. Inappropriate neutrophil activation has also been implicated in venous disease.

When polymorphonuclear leukocytes ("PMN") are stimulated with an agonist during an immunological response, the PMN exhibit a transient rise in intracellular free-calcium levels, attributable to a release of calcium from internal stores such as those within the endoplasmic reticulum. For example, after exposure to a chemoattractant, secretagogue, or activating agonist, neutrophils exhibit a rise in intracellular free-calcium levels. This rise in intracellular free-calcium is important for the subsequent activity of the PMN in the immunological response. Current models indicate that after this transient rise in intracellular free-calcium levels, neutrophils subsequently return to homeostasis and intracellular free-calcium levels return to a normal state by a process that involves $Ca^{++}$ resequestration back into intracellular stores, $Ca^{++}$ influx from the outside of the cell, and $Ca^{++}$ efflux into the external milieu.

Human milk has been shown to contain anti-inflammatory activity in both humans and in animal models (e.g., in chemically-induced colitis model and in a rat subcutaneous air pouch model of inflammation). Previous studies have shown that many pro-inflammatory functions of neutrophils are decreased by exposure to colostrum and/or human milk. For example, components in human milk can increase expression of DC11b, decrease expression of L-selectin and produce activated neutrophils. Intracellular free-calcium levels in neutrophils are related to neutrophil activity, and intracellular $Ca^{++}$ balance is critical for multiple normal neutrophil functions.

Studies have shown that inhibition of $Ca^{++}$ influx (either by a $Ca^{++}$ channel blocker or by suspending neutrophils in a $Ca^{++}$-free buffer) can decrease many pro-inflammatory neutrophil functions after physiologic stimulation, e.g., human neutrophil function inhibition by tolfenamic acid involves inhibition of $Ca^{++}$ influx. $N$-acetylsphingosine ($C_2$-ceramide) has been reported to inhibit neutrophil superoxide formation and calcium-influx. Drugs that block calcium channels are known to have an influence on the microbicidal function of human neutrophils and calcium channel blockers have been reported to influence polymorphonuclear and monocyte bactericidal and fungicidal activity. Selective suppression of PMN function has been attempted for therapeutic reasons using cytotoxic agents capable of suppressing PMN production by bone marrow to try to minimize inflammation induced injury to tissues. The marrow suppression has not been PMN-specific, however, and has lead to suppressed production of other blood elements with attendant side effects. Corticosteroid treatment has been used to suppress PMN function but such treatments also have a number of undesirable side effects. Selective suppression of PMN function in vivo has been reported to be achieved experimentally through the use of monoclonal antibodies directed against PMN integrins. This latter work suggests that suppression of PMN function may have application in the prevention/minimization of myocardial infarct extension and the amelioration of ischemia/reperfusion injury.

It has previously been reported that human milk inhibits PMN by reducing calcium stores, at least in part, through inhibition of the influx of extracellular calcium to PMN following agonist stimulation. Human milk exposure has also been reported to alter intracellular $Ca^{++}$ balance, cause cellular activation and suppress function of polymorphonuclear leukocytes. More recent work has reported that the ability of human milk to inhibit influx of extracellular calcium to PMN may be an effect that has significant specificity. Human milk exposure has been reported to have no inhibitory effect on extracellular calcium influx on rabbit smooth or striated muscle cells, on immature human myeloid cells or immature rat intestinal cells.

Summary

The present application relates to an extracellular calcium influx inhibition factor and a method for its purification from human milk. Human milk has broad anti-inflammatory properties, and human milk has been shown to inhibit cellular mediators of inflammation such as PMN or neutrophils. The activity of PMN and neutrophils has been correlated to intracellular free-calcium levels. Human milk may suppress the function of PMN or neutrophils by inhibiting the calcium-influx into the cells from external sources, when the PMN or neutrophils return to homeostasis after having been stimulated with an agonist. Other studies have suggested that human milk may also modify $Ca^{++}$ resequestration into intracellular stores, and $Ca^{++}$ efflux into the external milieu. Because intracellular calcium balance appears to be critical for multiple normal PMN functions, depletion of intracellular calcium stores could potentially provide an avenue for achieving anti-inflammatory effects through broad suppression of PMN function.

The present application provides a purified factor from human milk that is capable of inhibiting extracellular calcium influx in PMN. As employed herein, the terms "purified" and "purification" refers to compositions (and related methods) in which the relative amount of a designated component(s), such as a CaII factor, has been increased by removal of at least a portion of one or more impurities from the composition. Although these terms may refer to a process which produces a composition which includes 90 wt. % or more of the desired component(s), the terms "purified" and "purification" do not require or imply such a high degree of removal of other substances from the composition.

The central importance of $Ca^{++}$ metabolism to neutrophil function prompted the applicants to examine whether milk exposure altered neutrophil $Ca^{++}$ responses to a series of agonists and how the effects were achieved. The present application is based on the consideration that it should be possible to isolate a factor capable of inhibiting the influx of extracellular calcium into PMN (referred to herein as a "CaII factor") from human milk by using an assay that measures calcium-influx in PMN after stimulation with an agonist (e.g., formyl-methionyl-leucyl-phenylalanine ("fMLP")).

The present application provides a method of producing a purified factor from human milk that is capable of at least partially inhibiting extracellular calcium-influx in PMN (referred to herein as "calcium-influx inhibiting activity"). Preferably, the CaII factor is substantially purified, i.e., the CaII factor constitutes at least about 50 wt. % of the components having an apparent molecular weight of 1 kDa or greater which are present in a purified composition. When the PMN are treated with the purified CaII factor, the influx of external calcium into the PMN is inhibited. Therefore, upon further stimulation with an agonist and further release of calcium from internal stores, the PMN will experience a net depletion in calcium content and, as such, one or more functions of the PMN will be suppressed.

One embodiment provides a method of purifying a CaII factor derived from clarified human milk. Clarified milk has been substantially cleared of cellular debris and fat globules. This can be accomplished using standard methods such as centrifugation and/or filtration. The CaII factor can be purified by isolating a low molecular weight fraction from clarified milk such that the fraction contains components with an apparent molecular weight of not less than 1 kDa and not greater than 10 kDa. For example, the clarified milk can be processed to remove components having an apparent molecular weight greater than 10 kDa; and subsequently processed to remove components having an apparent molecular weight less than 1 kDa. In a preferred embodiment, the clarified milk can be processed to provide a low molecular weight fraction containing components having an apparent molecular weight no greater than 3.5 kDa and no less than 1 kDa. The low molecular weight fraction may be subsequently treated to remove lipid soluble components to provide an aqueous second fraction, which includes a component exhibiting calcium-influx inhibiting activity. This can be accomplished, for example, by extracting the first fraction with an organic solvent (e.g., a chloroform/methanol mixture) and retaining the aqueous phase.

For example, a purified CaII factor can be produced from clarified human milk by a method which includes (i) dialyzing clarified human milk through a 10 kDa cut-off membrane to collect a dialysate as a first fraction; (ii) dialyzing the first fraction through a 1 kDa cut-off membrane to isolate a retentate as a second fraction; and (iii) extracting the second fraction with an organic solvent capable of dissolving lipid-soluble components (e.g., chloroform/methanol mixture) to produce a third fraction which is an aqueous solution including a component that exhibits calcium-influx inhibiting activity.

The present application also provides a purified CaII factor derived from human milk. The CaII factor is capable of inhibiting the influx of extracellular calcium to PMN. The purified CaII factor is typically produced from clarified human milk. The CaII factor typically has an apparent molecular weight of not less than about 1 kda and not greater than about 10 kda. More suitably, it is believed that the CaII factor typically has an apparent molecular weight of not less than about 1 kda and not greater than about 3.5 kda. The CaII Factor is soluble in aqueous solution and is preferably purfied so that it is substantially free of lipid soluble material. It has been found that the factor is capable of retaining its extracellular calcium influx inhibiting activity ("CaII activity") after being heated for 10 minutes at 100°C. The CaII factor is also capable of retaining its CaII activity after being subjected to acidification to pH 2 for 10 minutes at 60°C or to alkalinization to pH 12 for 10 minutes at 25°C. The CaII factor does not produce a positive response in a Lowry assay and is capable of retaining its CaII activity after being treated with proteinase K at 1 $\mu$g/ml for 10 minutes at 37°C. As employed herein, the term "resistant to" refers to the a factor which continues to exhibit at least some degree of calcium-influx inhibiting activity after being subjected to the stated conditions. The CaII factor is also stable to freeze thaw treatment, i.e., the CaII factor exhibits calcium-influx inhibiting activity after being subjected to a freeze thaw treatment.

The present application also provides a method of inhibiting the influx of calcium into polymorphonuclear leukocytes which includes administering a CaII factor to the leukocytes.

Brief Description Of The Drawings

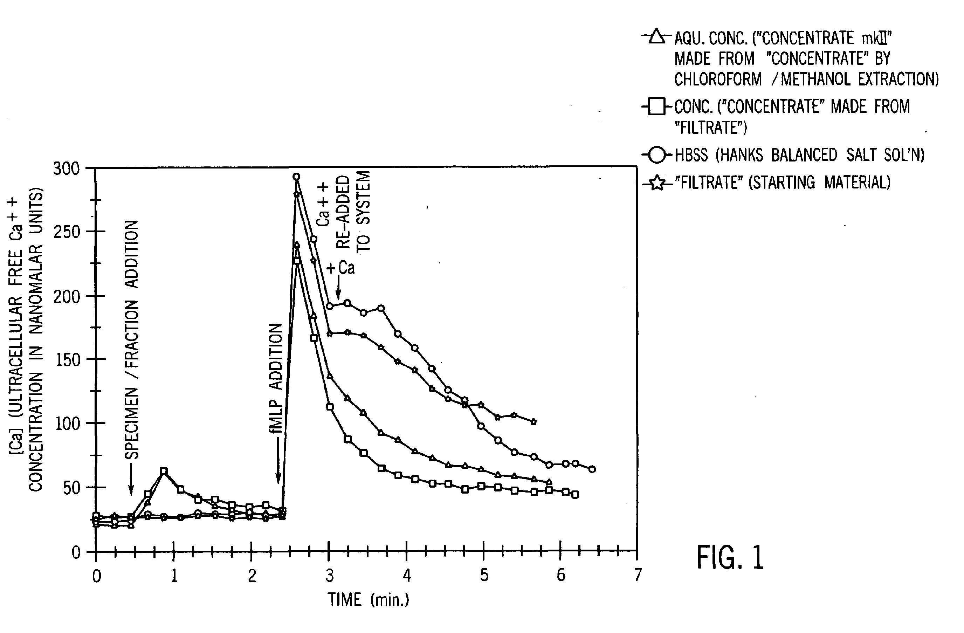

FIG. 1 shows a graph which depicts the inhibition of the influx of $Ca^{++}$ after stimulation with fMLP and addition of free $Ca^{++}$ into the media. The purified milk fraction that passes through a 10 kDa cut-off membrane is referred to as "Filtrate." The fraction of "Filtrate" that is retained by a 1 kDa Dalton cut-off membrane is referred to as "Concentrate" ("Conc. "). The "Aqu. Conc. "(alternatively referred to herein as "mkII") was made by extracting the "Conc" fraction with a chloroform/methanol mixture and collecting the aqueous phase.

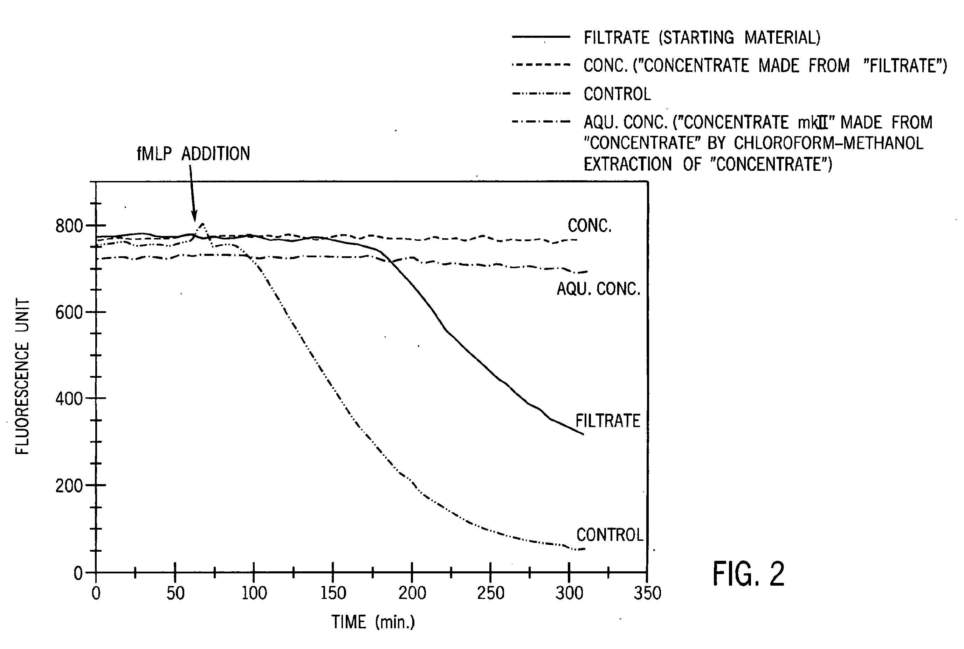

FIG. 2 shows a graph which depicts the results of a scopoletin assay after stimulation with *f*MLP. The "Filtrate," "Concentrate" ("Conc."), and "Aqu. Conc." are the same purified milk components referred to in the description of Fig. 1. In this assay, the loss of scopoletin fluorescence is an indicator of $H_2O_2$ production by PMN.

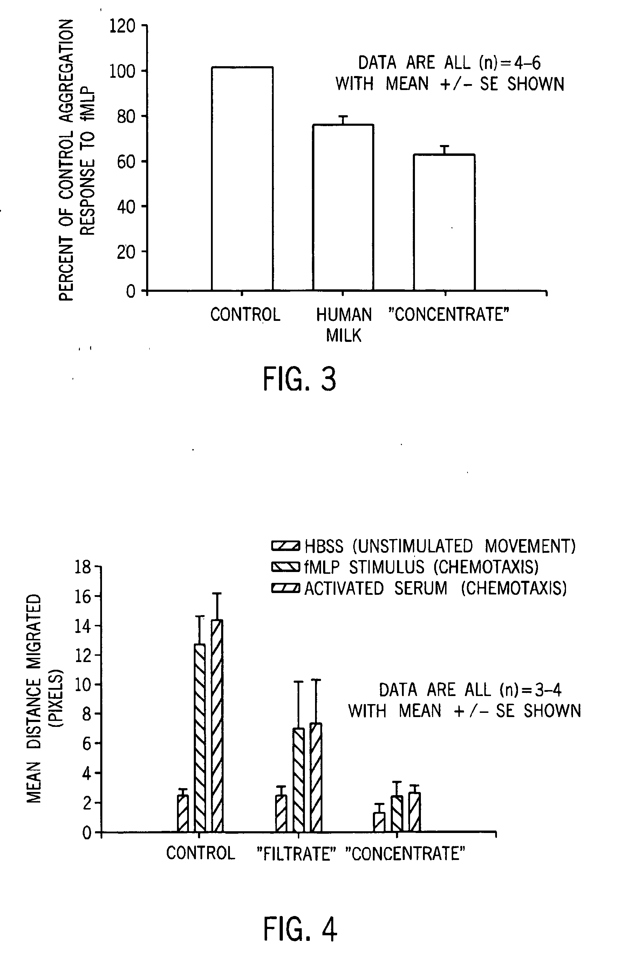

FIG. 3 shows a graph which depicts the effects of human milk and "Concentrate" ("Conc.") on PMN aggregation responses after stimulation with *f*MLP. "Conc." is the same purified milk component referred to in the description of Fig. 1.

FIG. 4 shows a graph which depicts the effects of "Filtrate" and "Concentrate" ("Conc. ") on PMN locomotion (either unstimulated or after stimulation with *f*MLP or activated serum). "Filtrate" and "Conc." are the same purified milk components referred to in the description of Fig. 1.

Detailed Description

This application relates to the isolation and/or purification of a factor from human milk that is capable of inhibiting the influx of calcium into PMN.

As used herein, the term "clarified human milk" refers to human milk that substantially has been cleared of cellular debris and fat globules, for example by centrifugation and/or filtration.

As used herein, the term "inflammation" refers to a localized protective response elicited by injury and/or destruction of tissues which serves to destroy, dilute or wall off an injurious agent and/or injured tissue and is characterized in the acute form by the classical sequences of pain, heat, redness, swelling, and loss of function. Inflamation histologically involves a complex series of events, including dilation of the arterioles, capillaries, and venules with increased permeability and blood flow, exudation of fluids including plasma proteins, and leukocyte migration into the inflammatory focus.

As used herein, the term "polymorphonuclear leukocytes" (or "PMN") refers to a representative class of white blood cells, alternatively called granulocytes, comprising neutrophils, eosinophils, and basophils.

As used herein, the term "HBSSw" refers to Hanks Balanced Saline Solution, supplemented with 10 mM $MgCl_2$ and 10 mM $CaCl_2$.

As used herein, the term "HBSSw/o" refers to Hanks Balanced Saline Solution, without 10 mM $MgCl_2$ and 10 mM $CaCl_2$.

As used herein, the term "*f*MLP" refers to a peptide of the sequence, formyl-methionyl-leucyl-phenylalanine.

As used herein, the term "calcium-influx" refers to displacement of calcium from the external media into a cell.

As used herein, the term "free-calcium" refers to intracellular calcium which is present in the cytosol and not retained within intracellular vesicles such as the endoplasmic reticulum.

One suitable method for isolating and purifying the factor from clarified human milk comprises the following steps:

  1. dialyzing the clarified human milk through a 10 kDa dialysis membrane and collecting the dialysate ("filtrate") as a first fraction, e.g., via overnight dialysis of the clarified human milk against phosphate buffered saline ("PBS") through a 10 kDa dialysis membrane;

  2. dialyzing the first fraction through a 1 kDa dialysis membrane and collecting the retentate ("concentrate") as a second fraction, e.g., via absorptive dialysis/concentration of the first fraction across a 1 kDa cut-off membrane;

  3. extracting the second fraction with an organic solvent (such as a mixture of chloroform and methanol) and collecting the resulting aqueous phase ("aqueous concentrate").

The presence and relative amount of the Call factor in a given fraction can be determined by performing a calcium-influx assay. The concentration of free calium in cells ("free Ca$^{++}$") can be measured by using a fura 2 probe (Molecular Probes, Eugene, OR). PMN, such as neutrophils, can be isolated as described in Boyum, A., "Isolation of mononuclear cells and granulocytes from human blood," *Scand. J. Clin. Invest.,* 97:77-89 (1968). The PMN or neutrophils, $5 \times 10^6$ PMN in HBSSw/o, can be labeled with fura-2 by exposure to $2\mu M$ fura-2AM (the methyl ester form of fura 2) for 45 min at 37°C, 5% $CO_2$, in total darkness darkness. After labeling, the cells can be washed twice by pelleting the cells, and resuspending the cells in a 15 ml volume of HBSSw/o. The concentration of cells can then be readjusted to $10 \times 10^6$/ml in HBSSw (with 10 mM Ca$^{++}$ and 10 mM Mg$^{++}$), and the emission of the fura 2 probe within the cells can be examined in an LS50B spectrofluorometer (Perkin Elmer Cetus, Norwalk, CT) at excitation wavelengths of 340 nm and 380 nm, and at an emission wavelength of 510 nm. When the fura 2 probe binds intracellular free Ca$^{++}$, it emits a higher intensity light when excited at 340 nm than 380 nm. This differential emission when the probe binds intracellular free Ca$^{++}$ allows the researcher to calculate the amount of free Ca$^{++}$ by using the equation:

$$\text{Free } Ca^{++} \text{ Conc.} = K_d((R-R_{min})/(R_{max} - R))Q$$

where $R$ is the ratio of the 340/380-nm fluorescence under the particular test conditions, $R_{max}$ is the ratio of the 340/380-nm fluorescence of cells treated with 0.1 % Triton X-100, $R_{min}$ is the ratio of the 340/380-nm fluorescence of cells treated with 0.1 % Triton X-100 and 20mM EGTA, "$Q$" is the ratio of 380-nm fluorescences under $Ca^{++}$-free/ $Ca^{++}$-saturated conditions, and $K_d$ is the disassociation constant for $Ca^{++}$ binding to fura 2 $(K_d = 145$ nM based on calibration curves). Following agonist stimulation, such as treating the PMN with 1$\mu$M fMLP, the calculated values of free $Ca^{++}$ can be used to produce a curve of free $Ca^{++}$ versus time, and the area under this curve is indicative of the free $Ca^{++}$ response to agonist.

The "CaII activity" in a given fraction can be measured by (1) treating PMNs with the purified fraction from human milk; (2) stimulating the PMNs with an agonist such as fMLP; (3) adding 10 mM $CaCl_2$ to the cell media approximately 30 seconds after stimulation with fMLP; and (4) measuring the rise, or lack thereof, of intracellular free $Ca^{++}$ versus time as compared to untreated cells after addition of the external 10 mM $CaCl_2$. CaII activity is defined as activity which reduces the area under the curve of intracellular free $Ca^{++}$ versus time relative to untreated cells after addition of 10 mM $CaCl_2$ to the external media. After stimulation with an agonist, PMNs exhibit a transient rise in intracellular free $Ca^{++}$ concentration, which then gradually declines (FIG. 1). After addition of external $Ca^{++}$, the intracellular free $Ca^{++}$ concentration again rises, except where the fraction contains a component exhibiting CaII activity (FIG. 1).

Purification Of The Factor

The present method of producing a purified CaII factor from human milk is exemplified by reference to the following description. This description is intended to illustrate but not limit the scope of the invention that has been set forth herein.

Mature human milk samples were collected from mothers more than four days after birth of the child by emptying the breast by manual expression or by electric breast pump. After collection, each sample was stored at refrigerator temperatures and transported to the laboratory within three hours. On arrival in the laboratory, milk samples were subjected to centrifugation (380 $\times$ g, 10 min, 4°C), and the acellular supernatant containing the cream and aqueous phase was collected, recombined, and stored at -70°C until used. Before experimental use, human milk samples were thawed and subjected to centrifugation (14,000 $\times$ g, 25 min, room temperature) using a tabletop microfuge to again provide an aqueous phase ("clarified human milk").

The clarified human milk was then dialyzed against PBS through either a 10 kDa cut-off membrane or a 3.5 kDa cut-off membrane. The dialysate ("Filtrate") was collected and "concentrated" by dialyzing a second time against PBS through a 1 kDa cut-off membrane and collecting the retentate ("Concentrate"). The Concentrate can then be extracted with a mixture of chloroform/methanol, and the aqueous fraction collected ("aqueous concentrate"). After extraction, such aqueous fractions ("Aq. Conc. ") retain CaII activity (see, FIG. 1). The resulting purified factor has essentially no protein content (as detectable via Lowry assay) and its CaII activity is proteinase K resistant. Exposure to the organic solvent during the extraction step does not appear to have any substantial effect on the CaII activity of the factor, e.g., no component of the Aq. Conc. having CaII activity appears to lose activity through denaturation resulting from exposure to the organic solvent.

As demonstrated by the scopoletin assay results shown in Figure 2, PMN exposed to the "Filtrate" produce hydrogen peroxide ("$H_2O_2$") after stimulation with fMLP as evidenced by the loss of scopoletin fluorescence. In contrast, exposure of fMLP stimulated PMN to either the "Concentrate" ("Conc.") or the "Aqu. Conc." did not result in a loss of scopoletin fluorescence, indicating a lack of substantial $H_2O_2$ production by PMN exposed to these fractions.

Characterization Of The Factor

The purified CaII factor has an apparent molecular weight of either 1-10 kDa (i.e., the factor is not retained by a 10 kDa dialysis membrane, but is retained by a 1 kDa membrane) or 1-3.5 kDa (i.e., the factor is not retained by a 3.5 kDa dialysis membrane, but is retained by a 1 kDa membrane) depending on the membrane employed in the first dialysis step. The factor partitions to the aqueous phase upon extraction with an organic solvent such as a chloroform/methanol mixture. The factor does not strongly bind to cationic or anionic resins, e.g., the present CaII factor does not bind to heparin sepharose or Dowex resin.

The CaII factor inhibits calcium-influx in PMN, after the PMN have been stimulated with an agonist, as measured by a reduction in the area under the curve of intracellular free $Ca^{++}$ concetration versus time for treated cells relative to untreated cells (see, e.g., Figure 1). As evidenced by the results shown in Figures 3 and 4, respectively, the CaII factor is also capable of inhibiting the aggregation of PMN exposed to fMLP and locomotion of PMN stimulated with either fMLP or activated serum. While unfractionated human milk has been reported to decrease the adherence of PMN to surfaces of tissue culture plates, the present CaII factor had no effect on PMN adherence (result not shown).

The following procedures set forth below can be employed to (a) determine the intracellular calcium concentration in PMN; (b) determine the effect of milk fractions on surface receptor shedding by PMN; (c) examine the effect of milk fractions on the shape of PMN exposed to fMLP; (d) examine the effect of milk fractions on PMN adherence to tissue culture plate wells; and (e) determine the effect of milk fractions on the aggregation of PMN exposed to fMLP.

## PMN Purification and Preparation:

PMN were purified from heparinized blood obtained from adult volunteer donors using a Hypaque-ficoll/dextran sedimentation method. The resulting cell preparations (typically >95% PMN) were adjusted to $10^7$ cells/ml in Hanks Balanced Salt Solution with $Ca^{++}$ and $Mg^{++}$ (HBSSw) and used in the experiments as described.

## Serum and Plasma Control Preparation:

Serum specimens were prepared from 6 fresh blood samples that were allowed to clot for 30 minutes at room temperature, rimmed, held in ice for 30-60 minutes to allow clot retraction, sedimented (500 x g, 10 minutes, 4°C) and the serum removed and frozen at -20°C until used. Plasma samples were prepared from 6 different heparinized blood samples by sedimentation (500 x g, 10 minutes, 4°C), filtration of the resulting platelet-rich plasmas through $0.4.\mu$ filters to remove contaminating platelets, and then storage at -20°C until use.

## Intracellular $Ca^{++}$ Measurements in PMN:

Purified PMN were transferred to Hanks Balanced Salt Solution without $Ca^{++}$ and $Mg^{++}$ (HBSSw/o), adjusted to $5 \times 10^6$ cells/ml, and combined with 2 $\mu$M fura2-AM (Molecular Probes, Inc, Eugene, OR) for 45 minutes at 37°C, 5% $CO_2$ as previously described (8). After fura2 labeling, the cells were washed twice (15 ml HBSSw/o), adjusted to $10^7$/ml in HBSSw, and placed in a LS-50B spectrofluorometer (Perkin Elmer Cetus, Norwalk, CT) with excitation wavelengths of 340 and 380 nm and an emission wavelength of 510 nm. After stabilization of the fluorescence readings, 0-9% milk was added to the cells and their response recorded. Subsequently, 1 $\mu$M fMLP was added and the resulting changes in fluorescence emission were recorded continuously. For experiments examining 10%, 50% and 100% milk exposures, fura2 labeled cells were resuspended in appropriately diluted milk, held at 37°C for 10 minutes, washed twice, readjusted to $10^7$/ml in HBSSw and placed in the spectrofluorometer. After equilibration to a stable baseline, 1 $\mu$M fMLP was added to the cells and their fluorescence emission recorded continuously over time. intracellular free $Ca^{++}$ concentrations were calculated as previously described, with $R_{max}$ and $R_{min}$ for each experimental condition based on the fluorescence observed after lysis of cells with 0.1 % Triton X-100 to calculate Rmax and chelation of $Ca^{++}$ with 20 mM EGTA to calculate $R_{min}$. The abrupt rise and slower fall in intracellular free $Ca^{++}$ concentration (the $Ca^{++}$ transient) that occurred after formyl peptide or milk exposures was quantitated by measuring the difference between the baseline level of intracellular free $Ca^{++}$ and the maximal height achieved post fMLP addition. In experiments examining the effects of multiple milk additions on fMLP-mobilized intracellular $Ca^{++}$ stores, 0-4 sequential additions of 3% milk to the fluorometer cuvette were followed by an fMLP addition, the height of the fMLP-induced $Ca^{++}$ transient was measured as above and expressed as a percentage of the control (no prior milk addition) fMLP-induced transient height.

## Surface Receptor Shedding Assays:

Purified PMN ($10^7$) were resuspended in 0.5 ml aliquots of milk/HBSSw dilutions and held at 37°C for 10 minutes. Milk controls comprised identical aliquots of diluted milk (without added cells), and plasma controls were plasma dilutions $\pm$ added cells. After incubation, cells were sedimented (5 minutes, 14,000 x g, room temperature), and these supernatants and their corresponding control milk dilutions were removed and frozen at -70°C until tested. For quantitation of receptor shedding, samples (with and without cells) were examined for their contents of sTNFRI and sTNFRII by EASIA (Medgenix Inc, Fleures, Belgium), and contents of sIL-1RII by ELISA (R&D Systems, Minneapolis, MN). The differences in soluble cytokine receptor content between samples with and without added cells were taken as the amounts of cytokine receptor shed during milk, purified factor, or plasma exposure. Experiments can be conducted using 2 different samples with each PMN preparation or a different PMN preparation with each sample.

## Shape Change Assays:

Purified PMN were adjusted to $2 \times 10^7$ cells/ml and exposed to HBSSw, 1 $\mu$M fMLP, milk, milk fractions, or serum dilutions, and held at 37°C for 5 minutes. After incubation, cells were fixed (formalin 10% v/v, 25°C, 30 minutes), sedimented, removed from the formalin and resuspended in cold HBSSw at $10^7$ cells/ml. Five $4\mu$l of cell suspension was combined 1:1 with 0.1 % crystal violet in water, placed under a coverslip and examined using an Olympus BH2 inverted microscope. Microscope images of cells were captured digitally (400x, Connectix Color QuickCam, Connectix, San Mateo, CA), saved as gray-scale files and imported into image analysis software (SigmaScan Pro, Jandel Scientific, San Rafael, CA) to measure the major and minor axis lengths (in pixels) of all cells captured in the digital image. From these measurements, the major axis:minor axis ratio was calculated for each cell (for round cells, major axis:minor axis=1). A minimum of 25 images (54-120 individual cells) from each experimental condition were collected and measured, the data pooled, and the mean axis ratio taken as the measure of cell shape for each sample examined. These mean values for the same dilutions of each sample were averaged, and the resulting values were taken as the measure of cell shape in the respective sample dilution conditions.

PMN Adherence Assays:

Purified PMN ($5 \times 10^6$/ml in HBSSw) in 1 ml aliquots were combined with milk or a milk fraction to achieve final sample concentrations from 0% to 50% in 2 ml. Half milliliter volumes of this cell suspension were placed in duplicate wells on 24 well tissue culture plates and 20 ng/ml (final) phorbol myristate acetate (PMA) was added to one of the duplicate wells. The culture plates were held at 37°C, 5% $CO_2$, for 15 minutes, then each well was washed gently x2 with HBSSw to remove non-adherent cells, drained, and 0.5 ml of HTAB buffer (0.5% hexadecyltrimethyl ammonium bromide in 50 mM potassium phosphate, pH 6.0) was added to each well (25°C, 15 minutes) to solubilize the adherent cells. After incubation, the HTAB buffer was removed and frozen at -70°C until assayed for peroxidase activity. To calibrate the assay, an aliquot of $2.5 \times 10^6$ PMN was sedimented, the cell pellet solubilized in HTAB buffer, and the peroxidase activity of this sample taken to represent the activity present in $2.5 \times 10^6$ cells. For peroxidase activity determinations, HTAB buffer supernates were thawed, 0.1 ml was combined with 2.9 ml of assay buffer (0.167 mg O-dianisodine HCl/ml and 0.0005 % $H_2O_2$ in 50 mM potassium phosphate buffer, pH 6.0) and the brown color development followed at $OD_{460}$ using a lambda-6 spectrophotometer (Perkin Elmer Cetus, Norwalk CT). Readings of color intensity were recorded every 10 second for 90 seconds, and the $\Delta OD_{460}$ over the final 60 seconds was taken as the measure of peroxidase activity in the sample. The observed $\Delta OD_{460}$ for each experimental sample was divided by the $\Delta OD_{460}$ observed with the standard representing $2.5 \times 10^6$ cells to convert the data to the percentage of cells adhering to plastic in each condition.

PMN Aggregation Assays:

Freshly purified PMN ($2 \times 10^7$/ml in HBSSw) were combined 1:1 with milk or a milk fraction to achieve the desired final sample concentrations and placed in a Chrono-Log model 560 platelet aggregometer at 37°C with continuous stirring at 500 rpm. From one experiment to the next, the order in which different sample concentrations were examined was reversed to avoid systematic artifacts due to cell aging during each experiment. During temperature equilibration, the upper and lower limits of light transmission were set at $8 \times 10^6$ and $10 \times 10^8$ cells/ml respectively, and fMLP (1 $\mu$M final) was added to the cell suspension to induce aggregation. The light transmission of aggregating cells was recorded for 3 minutes following fMLP addition, and the hard copy aggregation curve tracings were converted to digital images using a flat bed scanner. The resulting images were analyzed (SigmaScan Pro, Jandel Scientific, San Rafael, CA) to quantitate the 3 minute area under the aggregation curve (in $pixel^2$) as the measure of aggregation responses in each condition. Control experiments examining the effects of 1%, 4%, 10 %, 25% and 50 % serum on PMN aggregation responses to 1$\mu$M fMLP showed that none of these serum concentrations had any suppressive effect on PMN aggregation.

The invention has been described with reference to various specific and illustrative embodiments and techniques. However, it should be understood that many variations and modifications may be made while remaining within the spirit and scope of the invention.

Cross Reference To Other Applications

This application claims priority of U.S. Provisional Application Serial No. 60/322,057, filed on September 12, 2001, the disclosure of which is herein incorporated by reference.

**Claims**

1. A method of purifying a factor derived from clarified human milk comprising:

    (i) isolating a first fraction from clarified human milk wherein said first fraction contains components with an apparent molecular weight of not less than about 1 kDa and not greater than about 10 kDa;

(ii) removing lipid soluble components from said first fraction to provide a second fraction which includes a component exhibiting calcium-influx inhibiting activity.

2. The method of claim 1, wherein said calcium-influx inhibiting activity is further purified from said second fraction by isolating the factor based on hydrophobicity.

3. The method of claim 1 or claim 2, wherein removing lipid soluble components comprises extracting the first fraction with an organic solvent and retaining the aqueous phase.

4. A method of purifying a factor derived from human milk comprising:

(i) dialyzing clarified human milk through a 10 kDa cut-off membrane to provide a dialysate as a first fraction;
(ii) dialyzing said first fraction through a 1 kDa cut-off membrane to isolate a retentate as a second fraction;
(iii) extracting said second fraction with a solvent capable of dissolving lipid-soluble components to give a third fraction which includes a component exhibiting calcium-influx inhibiting activity.

5. The method of claim 4, wherein said calcium-influx inhibiting activity is further purified from said third fraction by isolating the factor based on hydrophobicity.

6. A purified factor derived from human milk produced by the method of any preceding claim.

7. A purified factor derived from human milk:

wherein said factor has an apparent molecular weight of not less than about 1 kDa and not greater than about 10 kDa;
said factor is soluble in aqueous solution and is substantially free of lipid soluble material; and
said factor exhibits calcium-influx inhibiting activity.

8. The factor of claim 7, wherein said factor is resistant to treatment with heat for 10 minutes at 100°C.

9. The factor of claim 7 or claim 8, wherein said factor is resistant to acidification to pH 2 for 10 minutes at 60°C.

10. The factor of claim 7, 8 or 9, wherein said factor is resistant to alkalinization to pH 12 for 10 minutes at 25°C.

11. The factor of any one of claims 7 to 10, wherein said factor is resistant to treatment with proteinase K at 1 $\mu$g/ml for 10 minutes at 37°C.

12. The factor of any one of claims 7 to 11, wherein said factor is stable to freeze thaw treatment.

13. The factor of any one of claims 7 to 12, wherein said factor does not produce a positive response in a Lowry assay.

14. The factor of any one of claims 7 to 13, wherein said factor has an apparent molecular weight no more than about 3.5 kDa.

15. An *in vitro* method of inhibiting the influx of calcium into polymorphonuclear leukocytes comprising administering a factor as claimed in claim 6.

16. The use of a factor as claimed in any one of claims 6 to 14 in the manufacture of a medicament for decreasing inflammatory responses arising from the influx of calcium into polymorphonuclear leukocytes.

**Patentansprüche**

1. Verfahren zum Reinigen eines Faktors, der von geklärter menschlicher Milch abgeleitet ist, welches aufweist:

(i) Isolieren einer ersten Fraktion der geklärten menschlichen Milch, wobei besagte erste Fraktion Komponenten mit einem scheinbaren Molekulargewicht von nicht weniger als etwa 1 kDa und nicht mehr als etwa 10 kDa enthält;

(ii) Entfernen von Lipid-löslichen Komponenten von der besagten ersten Fraktion, um eine zweite Fraktion bereitzustellen, die eine Komponente enthält, die eine Kalzium-Einstrom-inhibierende Aktivität zeigt.

2. Verfahren nach Anspruch 1, wobei besagte Kalzium-Einstrom-inhibierende Aktivität weiters aus besagter zweiter Fraktion durch Isolieren des Faktors basierend auf Hydrophobizität gereinigt wird.

3. Verfahren nach Anspruch 1 oder Anspruch 2, wobei das Entfernen der Lipid-löslichen Komponenten das Extrahieren der ersten Fraktion mit einem organischen Lösungsmittel und Zurückhalten der wässrigen Phase aufweist.

4. Verfahren zum Reinigen eines von menschlicher Milch abgeleiteten Faktors, welcher, welches aufweist:

(i) Dialysieren der geklärten menschlichen Milch durch eine Membran mit einer Ausschlussgrenze von 10 kDa, um ein Dialysat als eine erste Fraktion bereitzustellen;
(ii) Dialysieren besagter ersten Fraktion durch eine Membran mit einer Ausschlussgrenze von 1 kDa, um ein Retentat als eine zweite Fraktion zu isolieren;
(iii) Extrahieren der besagten zweiten Fraktion mit einem Lösungsmittel, welches in der Lage ist, die Lipid-löslichen Komponenten zu lösen, um eine dritte Fraktion zu ergeben, die eine Komponente enthält, die eine Kalzium-Einstrom-inhibierende Aktivität zeigt.

5. Verfahren nach Anspruch 4, wobei die besagte Kalzium-Einstrom-inhibierende Aktivität weiters aus besagter dritter Fraktion durch Isolieren des Faktors basierend auf Hydrophobizität gereinigt wird.

6. Ein aus humaner Milch stammender gereinigter Faktor der durch ein Verfahren nach einem der vorangehenden Ansprüchen hergestellt ist.

7. Ein aus humaner Milch stammender gereinigter Faktor:

wobei besagter Faktor ein scheinbares Molekulargewicht von nicht weniger als etwa 1 kDa und nicht mehr als etwa 10 kDa aufweist;
besagter Faktor in einer wässrigen Lösung löslich ist und im Wesentlichen frei von Lipidlöslichem Material ist; und
besagter Faktor eine Kalzium-Einstrom-inhibierende Aktivität zeigt.

8. Faktor nach Anspruch 7, wobei besagter Faktor gegenüber einer Wärmebehandlung für 10 Minuten bei 100°C resistent ist.

9. Faktor nach Anspruch 7 oder Anspruch 8, wobei besagter Faktor gegenüber einer Ansäuerung auf pH 2 für 10 Minuten bei 60°C resistent ist.

10. Faktor nach Anspruch 7, 8 oder 9, wobei besagter Faktor gegenüber einer Alkanisierung auf pH 12 für 10 Minuten bei 25°C resistent ist.

11. Faktor nach einem der Ansprüche 7 bis 10, wobei besagter Faktor gegenüber einer Behandlung mit Proteinase K bei 1 $\mu$g/ml für 10 Minuten bei 37°C resistent ist

12. Faktor nach einem der Ansprüche 7 bis 11, wobei besagter Faktor gegenüber einer Gefrier/Auftau-Behandlung stabil ist.

13. Faktor nach einem der Ansprüche 7 bis 12, wobei besagter Faktor keine positive Antwort in einer Lowry-Untersuchung erzeugt.

14. Faktor nach einem der Ansprüche 7 bis 13, wobei besagter Faktor ein scheinbares Molekulargewicht von nicht mehr als etwa 3,5 kDa aufweist.

15. In vitro Verfahren zum Inhibieren des Einstroms von Kalzium in polymorphonukleäre Leukozyten, welches das Verabreichen eins Faktors nach Anspruch 6 aufweist.

16. Verwendung eines Faktors nach einem der Ansprüche 6 bis 14 bei der Herstellung eines Medikamentes für das Verringern einer entzündlichen Reaktion, die durch den Einstrom von Kalzium in polymorphonukleäre Leukozyten entsteht.

**Revendications**

1. Procédé de purification d'un facteur dérivé de lait humain clarifié, comprenant :

   (i) l'isolement d'une première fraction du lait humain clarifié dans lequel ladite première fraction contient des composants d'un poids moléculaire apparent d'au minimum environ 1 kDa et d'au maximum environ 10 kDa;
   (ii) l'élimination des composants lipidiques solubles de ladite première fraction pour procurer une seconde fraction qui inclut un composant présentant une activité d'inhibition de l'influx de calcium.

2. Procédé selon la revendication 1, dans lequel ladite activité d'inhibition de l'influx de calcium est encore purifiée de ladite seconde fraction en isolant le facteur sur la base de l'hydrophobicité.

3. Procédé selon la revendication 1 ou la revendication 2, dans lequel l'élimination des composants lipidiques solubles comprend l'extraction de la première fraction avec un solvant organique et la rétention de la phase aqueuse.

4. Procédé de purification d'un facteur dérivé de lait humain comprenant :

   (i) la dialyse du lait humain clarifié à travers une membrane d'un seuil de 10 kDa pour procurer un dialysat comme première fraction;
   (ii) la dialyse de ladite première fraction à travers une membrane d'un seuil de 1 kDa pour isoler un rétentat comme seconde fraction;
   (iii) l'extraction de ladite seconde fraction avec un solvant capable de dissoudre les composants lipidiques solubles pour donner une troisième fraction qui inclut un composant présentant une activité d'inhibition de l'influx de calcium.

5. Procédé selon la revendication 4, dans lequel ladite activité d'inhibition de l'influx de calcium est encore purifiée de ladite troisième fraction en isolant le facteur sur la base de l'hydrophobicité.

6. Facteur purifié dérivé du lait humain produit par le procédé selon l'une quelconque des revendications précédentes.

7. Facteur purifié dérivé de lait humain, dans lequel :

   ledit facteur a un poids moléculaire apparent d'au minimum environ 1 kDa et d'au maximum environ 10 kDa;
   ledit facteur est soluble en solution aqueuse et est en substance dénué de matériel liposoluble; et
   ledit facteur présente une activité d'inhibition de l'influx de calcium.

8. Facteur selon la revendication 7, dans lequel ledit facteur est résistant au traitement par la chaleur pendant 10 minutes à 100°C.

9. Facteur selon la revendication 7 ou la revendication 8, dans lequel ledit facteur est résistant à l'acidification à pH 2 pendant 10 minutes à 60°C.

10. Facteur selon la revendication 7, 8 ou 9, dans lequel ledit facteur est résistant à l'alcalinisation à pH 12 pendant 10 minutes à 25°C.

11. Facteur selon l'une quelconque des revendications 7 à 10, dans lequel ledit facteur est résistant au traitement par la protéase K à 1 $\mu$g/ml pendant 10 minutes à 37°C.

12. Facteur selon l'une quelconque des revendications 7 à 11, dans lequel ledit facteur est stable au traitement par congélation-décongélation.

13. Facteur selon l'une quelconque des revendications 7 à 12, dans lequel ledit facteur ne produit pas de réponse positive à un dosage de Lowry.

**14.** Facteur selon l'une quelconque des revendications 7 à 13, dans lequel ledit facteur a un poids moléculaire apparent d'au maximum environ 3,5 kDa.

**15.** Procédé *in vitro* pour inhiber l'influx de calcium dans des leukocytes polymorphonucléaires comprenant l'administration d'un facteur selon la revendication 6.

**16.** Utilisation d'un facteur selon l'une quelconque des revendications 6 à 14 dans la fabrication d'un médicament pour diminuer les réponses inflammatoires découlant de l'influx de calcium dans les leukocytes polymorphonucléaires.

-△-AQU. CONC. ("CONCENTRATE mkII" MADE FROM "CONCENTRATE" BY CHLOROFORM / METHANOL EXTRACTION)

-□-CONC. ("CONCENTRATE" MADE FROM "FILTRATE")

-O-HBSS (HANKS BALANCED SALT SOL'N)

-☆-"FILTRATE" (STARTING MATERIAL)

[Ca] (ULTRACELLULAR FREE Ca++ CONCENTRATION IN NANOMALAR UNITS)

SPECIMEN / FRACTION ADDITION

fMLP ADDITION

Ca++ RE-ADDED TO SYSTEM

+Ca

TIME (min.)

FIG. 1

FIG. 2

FILTRATE (STARTING MATERIAL)
CONC. ("CONCENTRATE MADE FROM "FILTRATE")
CONTROL
AQU. CONC. ("CONCENTRATE mkII" MADE FROM "CONCENTRATE" BY CHLOROFORM-METHANOL EXTRACTION OF "CONCENTRATE")

FIG. 3

FIG. 4